# DEMANDE DE BREVET EUROPEEN

(11) **EP 0 799 608 A1**
(43) Date de publication de la demande: **08.10.1997**
(21) Numéro de dépôt: 97400651.2
(22) Date de dépôt: 24.03.1997
(51) Int. Cl.: A61F 5/448

(54) **Raccord pour appareillage pour d'ostomie**

(30) Priorité: 01.04.1996 FR 9604048
(71) Demandeur: B. BRAUN BIOTROL, 92107 Boulogne Billancourt (FR)
(72) Inventeur: Holtermann, Henri, 64500 Saint-Jean-De-Luz (FR)
(74) Mandataire: Orès, Bernard

(57) **Abrégé**

La présente Invention se rapporte à un raccord pour appareillage pour stomie. Ce raccord comprenant deux éléments (10, 30). Le premier élément (10) comporte une bague (11) ayant un contour interne (12) et un contour externe (13), tandis que le deuxième élément (30) comporte, sur une embase (31) qu'il présente et qui est munie d'une ouverture centrale, un premier logement (44) de formes et de dimensions conjuguées à celles d'une partie du contour interne (12) de la bague (11) du premier élément (10) et formant une gouttière de maintien étanche de ladite partie dudit contour interne, cette gouttière étant dirigée vers l'extérieur de l'embase (31), et un deuxième logement (45) de formes et de dimensions conjuguées à celles d'une partie du contour externe (13) de la bague (11) du premier élément (10) et formant une gouttière de maintien étanche de ladite partie dudit contour externe, cette gouttière étant dirigée vers l'ouverture de l'embase.

Applications : stomies et drainages chirurgicaux.

## Description

La présente Invention se rapporte à un raccord propre à entrer dans la constitution d'un appareillage pour stomie tel que, par exemple, un appareillage pour iléostomie, colostomie, urostomie ou pour le drainage chirurgical d'espaces ou de cavités intra-abdominales du type drainage péritonéal ou sous-péritonéal, ainsi qu'à un appareillage pour stomie comprenant un tel raccord.

De nombreux appareillages ont déjà été proposés pour assurer le recueil des matières corporelles (fèces, urine, ...) chez des personnes ayant subi une intervention chirurgicale du type stomie. Parmi ces appareillages, on distingue essentiellement :
- des appareillages qui sont constitués par une poche munie d'un protecteur cutané portant un adhésif sensible à la pression et qui est destinée à être fixée sur la peau de l'utilisateur par l'intermédiaire de ce seul protecteur cutané ; et
- des appareillages dits "deux-pièces" qui comprennent une plaque frontale ou "porte-poches" destinée à être fixée sur la peau de l'utilisateur, par un patin adhésif qu'elle présente ou une ceinture, et une poche apte à être assujettie de manière amovible sur cette plaque frontale.

Parmi les nombreuses exigences auxquelles doit satisfaire un appareillage pour stomie deux-pièces, il y a lieu qu'il permette un assemblage et un désassemblage de la poche et du porte-poches aussi simples et aisés que possible pour que les stomisés, même lorsqu'ils sont âgés, puissent procéder eux-mêmes sans difficulté à la mise en place d'une poche sur le porte-poches, tout en assurant une fixation sûre de ces deux éléments et en garantissant une étanchéité parfaite de l'appareillage.

C'est la raison pour laquelle il a été proposé des appareillages deux pièces dans lesquels le dispositif d'assemblage de la poche et du porte-poches comprend, d'une part, deux éléments dont l'un est situé sur la poche tandis que l'autre est fixé sur le porte-poches, et qui sont propres à coopérer ensemble lors de la mise en place de la poche sur le porte-poches et à assurer, par la présence d'une lèvre ou d'un joint d'étanchéité ou par la conjugaison de leurs formes et/ou de leurs dimensions, l'étanchéité de l'appareillage, et, d'autre part, un organe du type bague circulaire qui est généralement associé à l'un des deux embouts et qui assure, par des mécanismes différents selon les appareillages (effet de came, effet de serrage, ...) la solidarisation de la poche et du porte-poches. De tels appareillages sont décrits par exemple dans EP-A-255 310 et dans WO 91/01118.

Il en résulte que ces appareillages de stomie, bien qu'appartenant au type des appareillages deux-pièces, comprennent, pour l'assemblage de la poche sur le porte-poches, au moins trois éléments, ce qui en grève notablement le coût de production, tant au niveau de la réalisation des différentes pièces entrant dans la constitution de l'appareillage que de l'assemblage de ces pièces.

Le problème se pose donc de fournir un raccord permettant d'assembler un porte-poches et une poche de stomie qui, tout en satisfaisant aux différentes exigences requises pour un tel raccord, à savoir de maniabilité et de simplicité d'utilisation, de sécurité de fixation, de garantie d'étanchéité, d'adaptabilité aux différentes morphologies et de discrétion, ..., ne soit constitué que par deux éléments de manière à optimiser son coût de production.

On connaît par FR-A-2 387 643 ou EP-A-0 171 255, des appareillages deux-pièces dans lesquels le dispositif d'assemblage du porte-poches et de la poche est constitué par deux embouts, situés respectivement sur le porte-poches et sur la poche et qui sont conçus pour que l'emboîtement d'un embout dans l'autre, sous l'effet d'une poussée ou d'une pression appliquée par l'utilisateur, assure simultanément l'étanchéité de l'appareillage et la solidarisation de la poche sur le porte-poches.

Toutefois, ces dispositifs exigent qu'une pression importante soit exercée par l'utilisateur sur les deux embouts lors de la mise en place de la poche sur le porte-poches. Etant donné cependant que la zone péristomiale est sensible, voire parfois douloureuse, l'application d'une forte pression crée une gêne pour l'utilisateur en sorte que ces dispositifs ne sont pas vraiment satisfaisants.

Aussi, a-t'on proposé récemment dans US-A-5,139,492, un raccord constitué par deux anneaux à contour externe hexagonal qui présentent chacun un bord libre sur trois côtés consécutifs et un rabat de forme et de dimensions conjuguées à celles de ce bord libre sur les trois autres côtés. Ces anneaux sont destinés à être montés "tête-bêche", l'un sur le porte-poches et l'autre, sur la poche. La fixation de cette dernière résulte d'un engagement mutuel des deux anneaux qui est obtenu en faisant glisser simultanément le bord libre de chaque anneau dans le rabat de l'anneau qui lui fait face. S'il est vrai que ce dispositif présente l'avantage de permettre une mise en place de la poche sur le porte-poches sans application de pression sur la zone péristomiale, cette mise en place apparaît toutefois difficile à réaliser, notamment pour des personnes âgées parfois malhabiles et l'étanchéité entre les deux anneaux du raccord semble douteuse au niveau des zones de contact des deux rabats. En outre, la forme hexagonale des deux anneaux interdit, que ce soit au moment de la fixation de la poche sur le porte-poches ou postérieurement à cette fixation, d'orienter à volonté la poche par rapport au porte-poches comme cela est souvent souhaité par les utilisateurs pour des questions de confort.

On a, par ailleurs, proposé dans EP-A-0 598 625 un raccord de stomie dans lequel l'élément fixé sur le porte-poches est formé de deux parties sensiblement planes qui comportent, pour la première, une tubulure adaptée à coopérer à étanchéité avec un collet assujetti sur la poche et, pour la deuxième, une ouverture ayant une dimension sensiblement égale à la dimension extérieure de ladite tubulure et autour de laquelle est disposée une glissière propre à recevoir le collet de la poche, lesdites première et deuxième parties étant liées, à leurs parties inférieures, par une charnière et étant munies de moyens permettant de les solidariser l'une à l'autre à leurs parties supérieures. La fixation de la poche sur le porte-poches est obtenue en glissant le collet de la poche dans ladite glissière puis en rabattant la deuxième partie de l'élément du porte-poches sur la première, ce qui amène la tubulure à pénétrer dans le collet de la poche. Ce raccord a l'avantage de permettre de fixer la poche sur le porte-poches sans exercer de pression sur la zone péristomiale et d'orienter à volonté la poche par rapport au porte-poches. Toutefois, il présente l'inconvénient d'être constitué par trois éléments - même si deux d'entre eux peuvent être moulés d'un seul tenant - dont la réalisation apparaît relativement complexe, avec notamment de très faibles tolérances dimensionnelles au niveau du collet, de la tubulure, de l'ouverture et de la glissière si l'on veut obtenir une étanchéité satisfaisante. Il nécessite en outre une certaine habileté pour insérer le collet de la poche dans la glissière du porte-poches ou, au contraire, retirer ce collet de cette glissière compte-tenu de surcroît que, dans ce dernier cas, la poche peut être pleine de déchets, ce qui le rend inadapté à une utilisation par des personnes âgées.

C'est, par conséquent, un but général de l'Invention que de fournir un raccord pour l'assemblage d'un porte-poches et d'une poche de stomie qui satisfasse toutes les conditions requises pour un tel raccord et, notamment, de simplicité d'utilisation et de maniabilité, de sécurité de fixation et d'étanchéité, et qui ne soit toutefois constitué que par deux éléments afin que son coût de production soit peu élevé.

C'est, aussi, un but de l'Invention que de fournir un tel raccord qui permette une fixation de la poche sur le porte-poches sans application d'aucune pression sur la stomie ou à son voisinage.

C'est, encore, un but de l'Invention que de fournir un tel raccord qui permette de modifier facilement et sans risque pour l'utilisateur la position relative de la poche sur le porte-poches.

Le problème est résolu dans un raccord pour appareillage pour stomie comprenant deux éléments, le premier élément comportant une bague ayant un contour interne et un contour externe, caractérisé en ce que le deuxième élément comporte, sur une embase qu'il présente et qui est munie d'une ouverture centrale, un premier logement de formes et de dimensions conjuguées à celles d'une partie du contour interne de la bague du premier élément et formant une gouttière de maintien étanche de ladite partie dudit contour interne, cette gouttière étant dirigée vers l'extérieur de l'embase, et un deuxième logement de formes et de dimensions conjuguées à celles d'une partie du contour externe de la bague du premier élément et formant une gouttière de maintien étanche de ladite partie dudit contour externe, cette gouttière étant dirigée vers l'ouverture de l'embase.

Selon un mode de réalisation préféré de l'Invention, au moins l'un des logements du deuxième élément est formé par l'embase de cet élément et par une paroi axiale érigée sur ladite embase et prolongée par un rebord radial.

Au besoin, la paroi axiale dudit logement comporte des évidements régulièrement espacés.

Avantageusement, ces évidements se prolongent sur toute la largeur du rebord radial de ladite paroi jusqu'au bord libre de ce rebord. En variante, ils se prolongent au plus sur une partie de la largeur du rebord radial de ladite paroi.

Selon un mode de réalisation préféré de l'Invention, les deux logements du deuxième élément sont formés chacun par l'embase de celui-ci et par une paroi axiale érigée sur ladite embase et prolongée par un rebord radial, et les parois axiales de ces logements sont disposées sur l'embase de part et d'autre de l'ouverture de cette embase.

Avantageusement, les parois axiales des logements du deuxième élément s'étendent sur l'embase de manière à ce qu'existent deux zones de recouvrement et dans lesquelles lesdites parois axiales sont disposées en vis-à-vis pour former un canal propre à recevoir intérieurement la bague.

Avantageusement, l'embase du deuxième élément présentant un bord interne et un bord externe, la paroi axiale du premier logement est érigée sur le bord interne de ladite embase tandis que la paroi axiale du deuxième logement est érigée sur le bord externe de ladite embase.

Avantageusement également, la largeur du rebord radial de chacune des parois axiales des logements du deuxième élément est inférieure ou égale à la moitié de la largeur de la bague du premier élément.

Selon un autre mode de réalisation préféré de l'Invention, la bague du premier élément comporte, sur sa face opposée à celle destinée à venir en contact de l'embase du deuxième élément, une nervure prolongée par une collerette radiale pour sa fixation sur l'appareillage pour stomie.

Selon une disposition avantageuse de l'Invention, la bague du premier élément est à section droite sensiblement rectangulaire.

Selon une autre disposition avantageuse de l'Invention, la bague du premier élément est à section droite sensiblement en forme de U et loge un joint torique.

Selon encore un autre mode de réalisation préféré de l'Invention, les contours interne et externe de la bague du premier élément comportent chacun une découpe concave qui ménage avec la face de cette bague destinée à être en contact avec l'embase de l'élément deux lèvres propres à assurer, par déformation élastique, l'étanchéité entre les deux éléments du raccord.

De manière particulièrement préférée, la bague du premier élément et l'embase du deuxième élément sont annulaires.

Avantageusement, au moins l'un des deux éléments du raccord est muni de moyens de préhension propres à faciliter l'assemblage de ces deux éléments.

Quel que soit le mode de réalisation, l'Invention prévoit complémentairement la présence, sur la collerette du premier élément et/ou l'embase du deuxième élément, d'un ou plusieurs plots propres à prévenir tout déplacement radial involontaire du premier élément par rapport au deuxième élément lorsque ceux-ci sont en condition d'assemblage.

L'Invention a également pour objet un appareillage pour stomie tel que, par exemple, un appareillage pour iléostomie, colostomie, urostomie ou pour le drainage chirurgical d'espaces ou de cavités intra-abdominales du type drainage péritonéal ou sous; péritonéal, qui comprend un porte-poches destiné à être fixé autour d'une ouverture artificielle du corps d'un utilisateur ainsi qu'une poche pour le recueil des matières corporelles propre à être assemblée de manière amovible au porte-poches, et qui est caractérisé en ce qu'il comprend un raccord tel que défini ci-dessus.

L'Invention sera mieux comprise au moyen de la description qui suit, faite à titre d'exemple et en référence aux dessins annexés dans lesquels :
- la Figure 1 est une vue en perspective montrant, distants l'un de l'autre et dans une première forme de réalisation, les deux éléments constitutifs d'un raccord pour appareillage pour stomie selon l'Invention ;
- la Figure 2 est une vue en coupe des deux éléments de la Figure 1, toujours distants l'un de l'autre et selon la ligne X-X pour le premier élément et la ligne Y-Y pour le deuxième élément ;
- la Figure 3 est une vue en coupe analogue à celle de la Figure 2, mais qui montre comment s'effectue l'assemblage des deux éléments du raccord ;
- la Figure 4 est une vue en coupe analogue à celle de la Figure 2, mais après assemblage des deux éléments du raccord ;
- la Figure 5 est une vue schématique en perspective d'une forme de réalisation d'un appareillage pour stomie selon l'Invention et qui est muni du raccord de la Figure 1 ;
- la Figure 6 est une vue partielle en coupe selon la ligne X-X analogue à celle de la Figure 2, mais pour une variante de la forme de réalisation du premier élément d'un raccord selon l'Invention telle que montrée sur la Figure 1 ;
- la Figure 7 est une vue en perspective pour encore une autre forme de réalisation du premier élément constitutif d'un raccord selon l'Invention ;
- la Figure 8A est une vue en coupe selon la ligne Z-Z de la Figure 7 ;
- la Figure 8B est une vue analogue à celle de la Figure 8A, mais pour une variante de réalisation ;
- la Figure 9 est une vue partielle en coupe d'une variante de la forme de réalisation du deuxième élément d'un raccord selon l'Invention telle que montrée sur la Figure 1 ;
- la Figure 10 est une vue en perspective d'une autre forme de réalisation du deuxième élément constitutif d'un raccord selon l'Invention ;
- la Figure 11 est une vue en perspective qui montre encore une autre forme de réalisation du deuxième élément constitutif d'un raccord selon l'Invention.

Sur les dessins, les éléments des différentes formes de réalisation qui sont équivalents ont reçu des numéros de référence identiques.

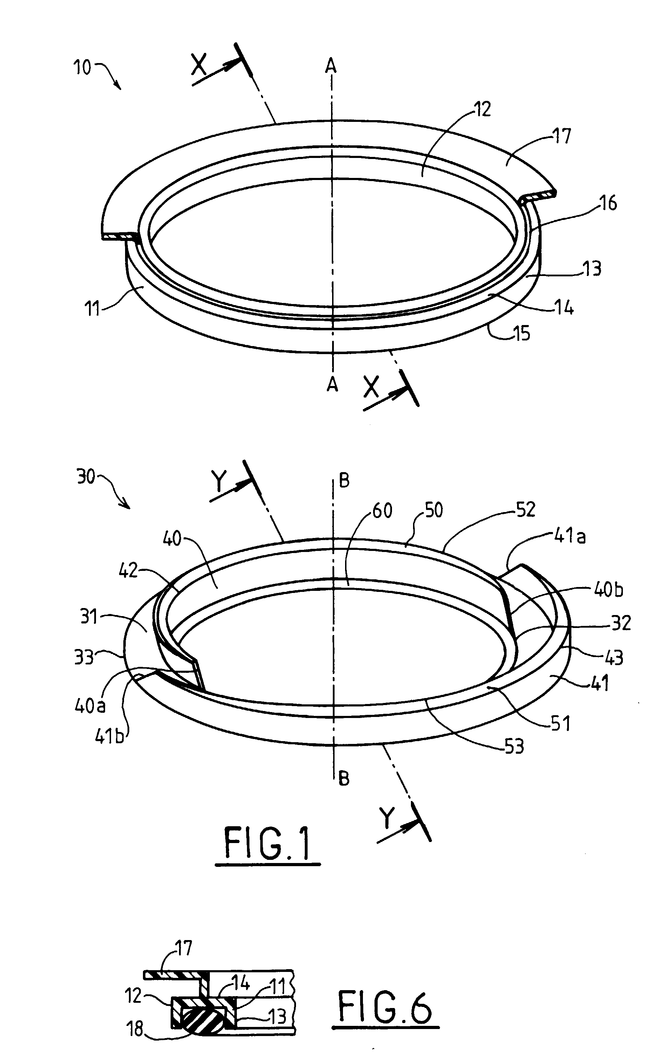

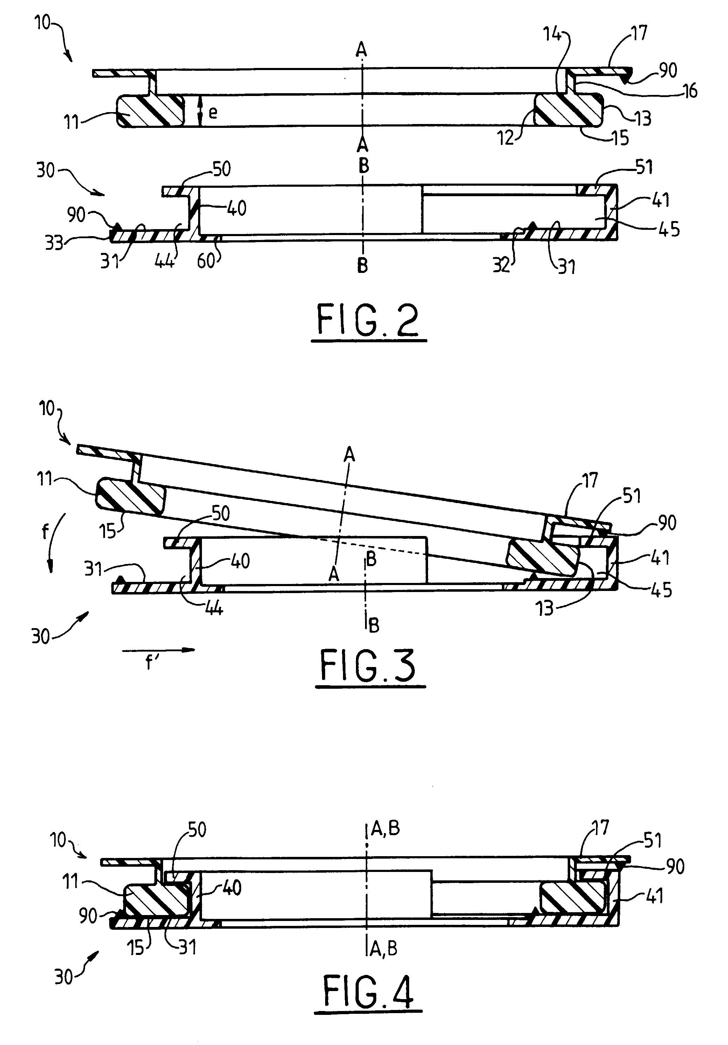

On se réfère tout d'abord aux Figures 1 et 2 qui montrent une première forme de réalisation d'un raccord pour appareillage pour stomie conforme à l'Invention et qui comprend deux éléments, respectivement 10 et 30, avantageusement réalisés en matière plastique et qui s'adaptent l'un à l'autre comme il sera explicité ci-après.

Le premier élément de ce raccord ou élément 10 comprend une bague annulaire 11, d'axe de révolution A et d'épaisseur e. Comme visible sur la coupe de la Figure 2, cette bague 11 est à section droite sensiblement rectangulaire avec un contour interne 12 et un contour externe 13 parallèles à l'axe A et deux faces 14 et 15 perpendiculaires à ce dernier.

Sur une nervure 16 qui occupe sensiblement la partie médiane de la face 14 de la bague annulaire 11, saille radialement vers l'extérieur de cette bague une collerette circulaire 17, co-axiale à cette dernière et parallèle à cette face 14. Cette collerette 17 qui est destinée à permettre la solidarisation, par soudure ou par collage ou analogue, de l'élément 10, soit sur le porte-poches, soit sur la poche d'un appareillage pour stomie, n'est volontairement représentée sur la Figure 1 que sur une portion de la face 14 de la bague annulaire 11 afin de mieux visualiser la structure de cette dernière.

Le deuxième élément du raccord ou élément 30 comprend une embase annulaire 31, d'axe de révolution B et qui présente un bord interne 32 et un bord externe 33. Sur cette embase 31, sont érigées deux parois 40 et 41, de même hauteur, co-axiales par rapport à l'axe B et diamétralement opposées par rapport à cet axe. La paroi 40 ou paroi interne est érigée sur le bord interne 32 de l'embase 31 tandis que la paroi 41 ou paroi externe est érigée sur le bord externe 33 de cette même embase.

Les parois 40 et 41 s'étendent toutes les deux sur plus de la moitié de la circonférence du bord de l'embase 11 qu'elles occupent de manière à ce que leurs extrémités 40a et 41b d'une part, et 40b et 41a d'autre part - lesquelles, comme représenté, peuvent se terminer en un biseau plus ou moins arrondi - soient situées dans deux secteurs angulaires diamétralement opposés et d'environ 10 à 25° chacun et ce, pour donner à l'élément 30, au niveau de ces extrémités, une tenue mécanique propre à éviter toute déformation de l'embase 31, par exemple du type pliure, dont il résulterait une altération de l'étanchéité entre les éléments 10 et 30 du raccord dans leur condition d'assemblage.

Par ailleurs, les parois 40 et 41 de l'élément 30 comportent chacune, sur leur bord, respectivement 42 et 43, opposé à celui qui est solidarisé à l'embase 31, un rebord, respectivement 50 et 51, qui est sensiblement parallèle à l'embase 31 et, ainsi, perpendiculaire comme cette dernière à l'axe B. Le rebord 50 s'étend vers l'extérieur de l'embase 31 tandis que le rebord 51 s'étend, au contraire, vers le centre de cette embase. Comme cela est visible sur la Figure 1, les rebords 50 et 51 présentent sensiblement la même largeur, mais celle-ci diminue au voisinage des extrémités des bords 42 et 43 de manière à ce que le bord libre, respectivement 52 et 53, des rebords 50 et 51 vienne rejoindre, au niveau de ces extrémités, le bord 42 pour le premier, et le bord 43 pour le second, en formant avec eux une pointe.

Les rebords 50 et 51 délimitent ainsi avec la paroi dont ils sont solidaires - paroi 40 pour le rebord 50 et paroi 41 pour le rebord 51 - et avec l'embase 31, deux logements 44 et 45 qui s'étendent tous deux sur plus de la moitié de la circonférence de l'embase 31 et qui sont dirigés, pour le premier, vers l'extérieur de cette embase et, pour le second, vers le centre de celle-ci en sorte qu'ils se rejoignent au niveau des extrémités 40a et 41b d'une part, et 40b et 41a d'autre part, des parois 40 et 41 en formant un canal dont le fond est constitué par l'embase 31 et dont les côtés sont constitués par les parois 40 et 41.

Conformément à l'Invention, les logements 44 et 45 sont destinés à loger la bague annulaire 11 de l'élément 10, dans la condition d'assemblage des deux éléments 10 et 30 du raccord, et à maintenir cette dernière solidaire grâce aux rebords 50 et 51 qui vont venir recouvrir respectivement une portion de son contour interne 12 et une portion de son contour externe 13.

Pour rendre possible cette réception tout en garantissant une étanchéité satisfaisante entre les éléments 10 et 30 dans leur condition d'assemblage - cette étanchéité étant prévue pour n'être assurée, dans la forme de réalisation décrite et représentée sur les Figures 1 et 2, que par la création de zones de contact surfacique entre d'une part, l'embase 31 et la face 15 de la bague annulaire 11 et entre, d'autre part, les parois 40 et 41 et une partie de même longueur qu'elles, respectivement du contour interne 12 et du contour externe 13 de cette bague annulaire - il convient que la distance séparant les bords 32 et 33 de l'embase 31 diminuée de l'épaisseur des parois 40 et 41 soit ajustée à la distance qui sépare les contours 12 et 13 de ladite bague annulaire 11. De manière similaire, il convient que la hauteur des parois 40 et 41 diminuée de l'épaisseur des rebords 50 et 51 soit ajustée à l'épaisseur e de la bague annulaire 11.

Comme visible sur les Figures 1 et 2, l'élément 30 comporte, en outre, une collerette circulaire 60 qui saille radialement du bord interne 32 de l'embase 31 vers le centre de cette embase et qui est destinée à permettre la fixation de cet élément, soit sur le porte-poches, soit sur la poche d'un appareillage pour stomie.

On se réfère à présent aux Figures 3 et 4 qui illustrent la façon dont les éléments 10 et 30 du raccord conforme à l'Invention sont assemblés l'un à l'autre. Pour réaliser cet assemblage, il convient en premier lieu de glisser la bague annulaire 11 de l'élément 10 dans le logement 45 de l'élément 30, suffisamment pour qu'une portion de son contour externe 13 soit recouverte par le rebord 51 et soit, ainsi, retenue par ce dernier mais sans toutefois la glisser jusqu'au fond de ce logement, fond qui est représenté par la paroi 41. Puis, on pivote la bague annulaire 11 dans le sens de la flèche f de manière à amener la portion de bague diamétralement opposée à celle qui est partiellement glissée dans le logement 45 au contact, par l'intermédiaire de sa face 15, de l'embase 31 et, ainsi, à mettre cette bague annulaire à plat sur l'élément 30. Il suffit alors de déplacer radialement la bague annulaire 11 par rapport à l'élément 30 dans le sens de la flèche f' jusqu'à ce que leurs axes de révolution respectifs A et B se confondent en un axe unique pour obtenir sa solidarisation sur ledit élément.

Dans cette condition, la bague annulaire 11 est logée pour partie dans le logement 44 de l'élément 30 et pour partie dans le logement 45 de ce même élément, avec sa face 15 qui est en contact avec l'embase 31, une portion de son contour interne 12 qui est en contact avec la paroi 40 et une portion de son contour externe 13 qui, elle, est en contact avec la paroi 41 - soit la création de trois zones de contact surfacique qui assurent l'étanchéité du raccord - et est retenue sur l'élément 30 grâce aux rebords 50 et 51 qui recouvrent une portion de même longueur qu'eux, respectivement du contour interne 12 et du contour externe 13 de cette bague.

L'assemblage des deux éléments 10 et 30 du raccord est donc extrêmement simple et ne nécessite aucune application de pression. En outre, si cet assemblage conduit bien à une solidarisation des deux éléments 10 et 30 propre à empêcher toute séparation involontaire de ceux-ci, il offre néanmoins la possibilité de tourner la bague annulaire 11 de l'élément 10, ce qui permet, lorsqu'un appareillage de stomie est équipé d'un tel raccord, d'orienter à volonté la poche de stomie par rapport au porte-poches.

Comme mentionné ci-avant, les deux éléments 10 et 30 d'un raccord conforme à l'Invention sont avantageusement réalisés en matière plastique et chaque élément est de préférence moulé d'un seul tenant dans une telle matière. Pour la forme de réalisation de ce raccord telle que décrite ci-dessus, il est avantageux de réaliser l'un des deux éléments et, notamment, l'élément 30 en un matériau plastique relativement rigide (d'une dureté de préférence comprise entre 66 et 86 Shore D) du type polyéthylène haute densité, polypropylène, polyamide, polyester thermoplastique, polyméthacrylate, ... et de réaliser l'autre élément en un matériau plastique qui est, au contraire, relativement souple (c'est à dire d'une dureté de préférence comprise entre 35 et 65 Shore D) du type polyéthylène basse densité, copolymère éthylène/acétate de vinyle, ...

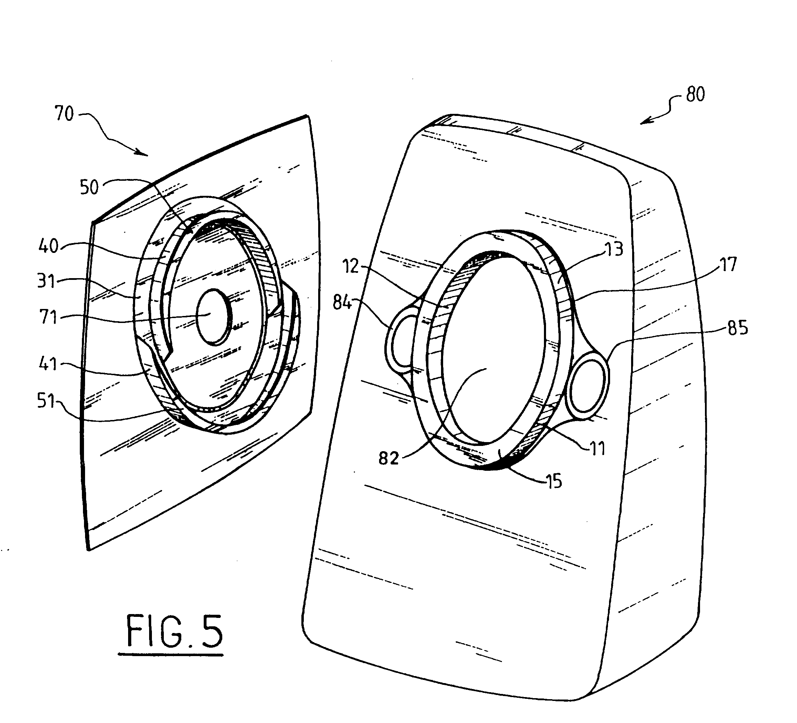

On se réfère à présent à la Figure 5 qui illustre de manière schématique une forme de réalisation d'un appareillage pour stomie qui est muni d'un raccord conforme à celui qui vient d'être décrit et qui est représenté sur les Figures 1 et 2.

Cet appareillage pour stomie comprend de façon en soi connue d'une part, un porte-poches 70 adapté à être fixé autour de la stomie d'un utilisateur et à permettre, par une ouverture centrale 71 qu'il présente, l'évacuation des matières corporelles de la stomie, et, d'autre part, une poche 80 propre à être assujettie de manière amovible au porte-poches 70 pour recueillir les matières corporelles ainsi évacuées au travers de l'ouverture 71 dudit porte-poches.

Dans la forme de réalisation de l'appareillage pour stomie qui est présenté sur la Figure 5, l'élément 30 du raccord est fixé sur le porte-poches 70 tandis que l'élément 10 de ce raccord est fixé sur la poche 80. Il est toutefois possible de réaliser un appareillage pour stomie dans lequel, à l'inverse, c'est le porte-poches qui est muni de l'élément 10 tandis que la poche est munie de l'élément 30. Dans ce cas, toutefois, il convient de solidariser l'élément 30 autour de l'ouverture 82 de la poche de telle sorte que les rebords 50 et 51 des parois 40 et 41 de cet élément soient orientés vers la partie inférieure de la poche.

Le fonctionnement de l'appareillage pour stomie tel que décrit et représenté sur la Figure 5 résulte de ce qui précède. Une fois le porte-poches 70 assujetti au corps de l'utilisateur, la poche 80 est présentée en regard du porte-poches et est fixée sur ce dernier en assemblant l'élément 10 et l'élément 30 exactement comme décrit ci-avant et représenté sur les Figures 3 et 4. Pour séparer la poche 80 du porte-poches 70, c'est un processus inverse qui est bien entendu mis en oeuvre.

La fixation de la poche 80 sur le porte-poches 70 ainsi que leur séparation peuvent être rendues encore plus faciles si on prévoit complémentairement, comme visible sur la Figure 5, la présence de deux oreilles 84 et 85 sur l'élément du raccord qui est solidarisé à la poche - en l'espèce l'élément 10 - ces oreilles étant diamétralement opposées et saillant radialement vers l'extérieur dudit élément et servant de moyens de préhension et de manipulation de ce dernier et, partant, de la poche qui en est solidaire. En outre, en munissant chacune d'elles d'un orifice comme représenté sur la Figure 5, les oreilles 84 et 85 peuvent également servir à renforcer le maintien de l'appareillage pour stomie sur le corps de l'utilisateur au moyen d'une ceinture.

Les oreilles 84 et 85 peuvent avantageusement prendre naissance sur le bord externe de la collerette 17 de l'élément 10 lorsque c'est celui-ci que l'on souhaite 1 munir de telles oreilles et sur le bord externe 33 de l'embase 31 de l'élément 30 lorsque c'est, au contraire, cet élément 30 que l'on souhaite munir de ces oreilles.

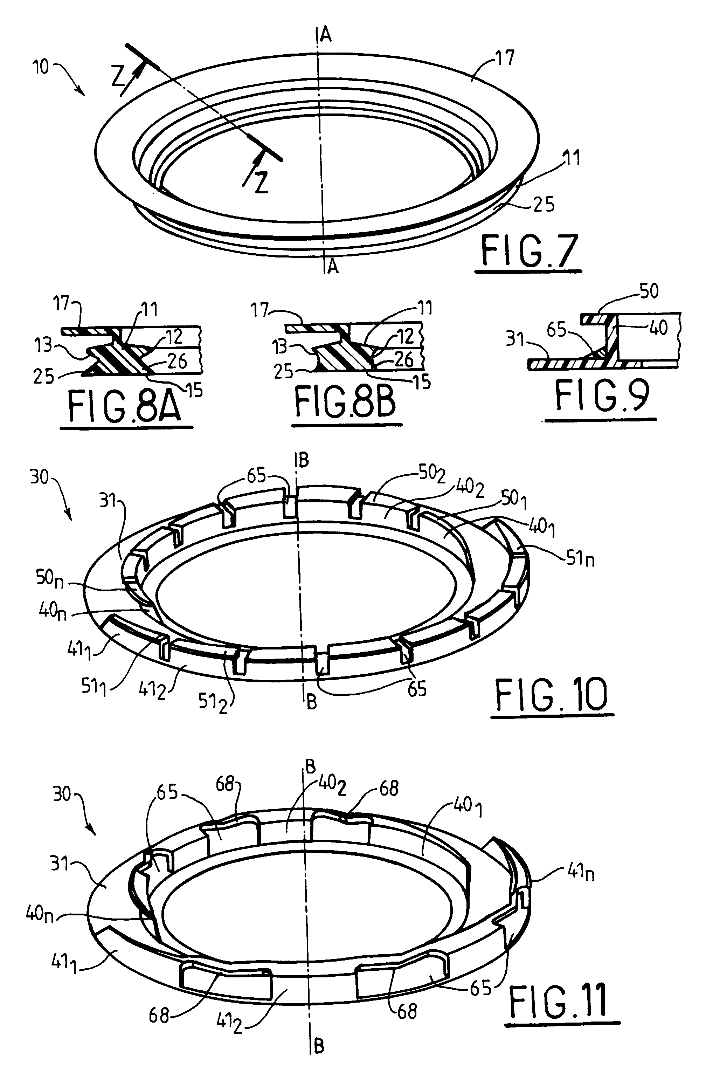

La variante montrée sur la coupe partielle de la Figure 6 prévoit que la bague annulaire 11 de l'élément 10 présente une section droite de forme générale en U et loge un joint torique 18, à section droite ovale ou ellipsoïde. Ce joint torique 18 qui est avantageusement réalisé en un matériau élastique tel qu'un élastomère thermoplastique, est propre à assurer, en s'écrasant sur l'embase 31, l'étanchéité entre les deux éléments 10 et 30 du raccord dans leur condition d'assemblage.

Les Figures 7, 8A et 8B illustrent une autre forme de réalisation de l'élément 10 du raccord conforme à l'Invention. Dans cette forme de réalisation, l'élément 10 est de préférence réalisé en un matériau plastique relativement rigide (d'une dureté de préférence comprise entre 66 et 86 Shore D). Comme dans la forme de réalisation représentée sur les Figures 1 et 2, il comprend une bague annulaire 11 munie sur la partie médiane de sa face 14 d'une nervure 16 d'où saille radialement vers l'extérieur une collerette 17 pour sa fixation sur le porte-poches ou la poche d'un appareillage pour stomie. Mais, à la différence de la forme de réalisation représentée sur les Figures 1 et 2, une découpe concave est ménagée dans chacun des contours interne 12 et externe 13 et accessoirement dans la face 15 de la bague annulaire 11 de manière à ce que ces contours forment avec cette face deux lèvres 25 et 26, lesquelles sont destinées à assurer, par déformation élastique, l'étanchéité entre les éléments 10 et 30 dans leur condition d'assemblage. Comme visible sur les Figures 8A et 8B qui montrent deux variantes possibles de cette forme de réalisation, ces découpes concaves peuvent être plus ou moins importantes et les lèvres d'étanchéité 25 et 26 ainsi constituées plus ou moins marquées. L'élément 10 ainsi réalisé est adapté à être assemblé à l'élément 30 tel que représenté sur les Figures 1 et 2. Toutefois, dans ce cas et comme visible sur la coupe partielle de la Figure 9, il est avantageux de prévoir, à la base de la paroi de l'élément 30 qui est érigée sur le bord interne de l'embase 31, à savoir la paroi 40, la présence d'une rondelle 65 à section droite triangulaire et dont la hauteur diminue de la partie médiane de ladite paroi en direction de ses extrémités. Cette rondelle 65 qui est avantageusement moulée d'un seul tenant avec l'embase 31 et la paroi 40, permet en effet, lorsque les éléments 10 et 30 sont assemblés, de compenser de manière sûre la diminution du diamètre interne de la bague annulaire 11 au niveau de la lèvre d'étanchéité 26, obligeant celle-ci à être en contact étroit avec l'embase 31 afin d'assurer l'étanchéité sous l'effet de l'écrasement de la partie 12 de la bague annulaire 11 entre le rebord 50 et l'embase 31.

On se réfère à présent à la Figure 10 qui montre une autre forme de réalisation du deuxième élément, c'est à dire de l'élément 30, du raccord conforme à l'Invention. Dans cette forme de réalisation, l'élément 30 qui est de préférence en un matériau plastique relativement rigide comprend, de manière analogue à celle des Figures 1 et 2, une embase annulaire 31, d'axe de révolution B et sur les bords de laquelle - bords interne 32 et externe 33 - sont érigées en vis-à-vis deux parois 40 et 41, co-axiales par rapport à l'axe B et parallèles à cet axe. Toutefois, à la différence de la forme de réalisation représentée sur les Figures 1 et 2, des évidements 65, régulièrement espacés angulairement et qui règnent sur des secteurs angulaires de quelques degrés, sont ménagés dans toute l'épaisseur des parois 40 et 41 en sorte que ces dernières sont divisées en une pluralité de secteurs circulaires, respectivement 40₁, 40₂, ..., 40ₙ et 41₁, 41₂, ..., 41ₙ, qui sont, sur la Figure 10, au nombre de 8 par paroi sans que cela soit toutefois limitatif. Ces secteurs circulaires sont munis, sur leur bord opposé à celui qui est solidarisé à l'embase 31, d'un rebord, respectivement 50₁, 50₂, ..., 50ₙ et 51₁, 51₂, ..., 51ₙ, sensiblement parallèle à l'embase 31, les rebords des secteurs 40₁, 40₂, ..., 40ₙ étant dirigés vers l'extérieur de cette embase tandis que ceux des secteurs 41₁, 41₂, ..., 41ₙ sont dirigés vers le centre de cette dernière. Cette configuration, si elle ne modifie pas dans leur principe ni la structure, ni la fonction de l'élément 30, permet de donner à cet élément une souplesse propre à lui conférer notamment une plus grande adaptabilité à l'anatomie de chacun des utilisateurs.

Dans encore une autre forme de réalisation de l'élément 30 qui est celle montrée sur la Figure 11, des évidements 65, régulièrement espacés angulairement, sont également ménagés dans l'épaisseur des parois 40 et 41 en sorte que, dans cette réalisation aussi, les parois 40 et 41 sont divisées en plusieurs secteurs circulaires, respectivement 40₁, 40₂, ..., 40ₙ et 41₁, 41₂, ..., 41ₙ. Mais, à la différence de la forme de réalisation montrée sur la Figure 10, d'une part, ces évidements 65 règnent sur des secteurs angulaires d'environ 10 à 30° chacun. D'autre part, les rebords de deux secteurs circulaires consécutifs d'une même paroi sont reliés par un pont de matière 68 sensiblement parallèle à l'embase 31 et qui s'étend dans le plan perpendiculaire à celui de l'axe B en formant un V dont la pointe est dirigée vers le centre de l'embase 31 pour les secteurs 40₁, 40₂, ..., 40ₙ et vers l'extérieur de cette embase pour les secteurs 41₁, 41₂, ..., 41ₙ. Cette configuration permet, tout en apportant de la souplesse à l'élément 30, une déformation harmonieuse des différents secteurs circulaires de cet élément.

L'élément 30, dans les formes de réalisation représentées sur les Figures 10 et 11, est adapté à être assemblé à l'élément 10 aussi bien dans ses formes de réalisation représentées sur les Figures 1, 2 et 6 que dans celles montrées sur les Figures 7, 8A et 8B, encore que, en pratique, on préfère l'assembler avec ces dernières formes de réalisation de l'élément 10.

Quelle que soit la forme de réalisation des éléments 10 et 30, il est avantageux de prévoir soit sur l'embase 31 de l'élément 30, en regard des parois 40 et 41 et à faible distance respectivement du bord externe 33 et du bord interne 32 de cette embase, soit sur la face de la collerette 17 de l'élément 10 qui se trouve en regard de la face 14 de la bague annulaire 11 et à faible distance du bord externe de cette collerette, soit éventuellement sur les deux - comme représenté sur les Figures 2 à 4 -, la présence d'un ou plusieurs plots 90 adaptés à empêcher, lorsque les deux éléments 10 et 30 sont assemblés l'un à l'autre, que l'élément 10 ne se déplace radialement par rapport à l'élément 30 (dans le sens contraire de celui indiqué par la flèche f') et, ainsi, que la bague annulaire 11 ne s'échappe des logements 44 et 45, ce qui entraînerait la désolidarisation des deux éléments 10 et 30 du raccord.

Bien entendu, la hauteur de ces plots 90 doit être choisie de manière à ce qu'ils puissent exercer leur fonction de blocage dans la condition d'assemblage des éléments 10 et 30 mais sans toutefois entraver l'assemblage et le désassemblage volontaires de ces éléments.

Ainsi que cela ressort de ce qui précède, l'Invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente Invention.

## Revendications

1. Raccord pour appareillage pour stomie comprenant deux éléments (10, 30), le premier élément (10) comportant une bague (11) ayant un contour interne (12) et un contour externe (13), caractérisé en ce que le deuxième élément (30) comporte, sur une embase (31) qu'il présente et qui est munie d'une ouverture centrale, un premier logement (44) de formes et de dimensions conjuguées à celles d'une partie du contour interne (12) de la bague (11) du premier élément (10) et formant une gouttière de maintien étanche de ladite partie dudit contour interne, cette gouttière étant dirigée vers l'extérieur de l'embase (31), et un deuxième logement (45) de formes et de dimensions conjuguées à celles d'une partie du contour externe (13) de la bague (11) du premier élément (10) et formant une gouttière de maintien étanche de ladite partie dudit contour externe, cette gouttière étant dirigée vers l'ouverture de l'embase.

2. Raccord selon la Revendication 1, caractérisé en ce qu'au moins l'un des logements (44, 45) du deuxième élément (30) est formé par l'embase (31) de cet élément et par une paroi axiale (40, 41) érigée sur ladite embase et prolongée par un rebord radial (50, 51).

3. Raccord selon la Revendication 2, caractérisé en ce que la paroi axiale (40, 41) dudit logement (44, 45) comporte des évidements (65) régulièrement espacés.

4. Raccord selon la Revendication 3, caractérisé en ce que les évidements (65) de la paroi axiale (40, 41) se prolongent sur toute la largeur du rebord radial (50, 51) de ladite paroi jusqu'au bord libre (42, 43) de ce rebord.

5. Raccord selon la Revendication 3, caractérisé en ce que les évidements (65) de la paroi axiale (40, 41) se prolongent au plus sur une partie de la largeur du rebord radial (50, 51) de ladite paroi.

6. Raccord selon l'une quelconque des Revendications 2 à 5, caractérisé en ce que les deux logements (44, 45) du deuxième élément sont formés chacun par l'embase (31) de celui-ci et par une paroi axiale (40, 41) érigée sur ladite embase et prolongée par un rebord radial (50, 51) et en ce que les parois axiales de ces logements sont disposées sur l'embase (31) de part et d'autre de l'ouverture de cette embase.

7. Raccord selon la Revendication 6, caractérisé en ce que les parois axiales (40, 41) des logements (44, 45) du deuxième élément (30) s'étendent sur l'embase (31) de manière à ce qu'existent deux zones de recouvrement et dans lesquelles lesdites parois axiales sont disposées en vis-à-vis pour former un canal propre à recevoir intérieurement la bague.

8. Raccord selon l'une quelconque des Revendications 2 à 7, caractérisé en ce que, l'embase (31) du deuxième élément présentant un bord interne (32) et un bord externe (33), la paroi axiale (40) du premier logement (44) est érigée sur le bord interne (32) de ladite embase tandis que la paroi axiale (41) du deuxième logement (45) est érigée sur le bord externe (33) de ladite embase.

9. Raccord selon l'une quelconque des Revendications 2 à 8, caractérisé en ce que la largeur du rebord radial (50, 51) de chacune des parois axiales (40, 41) des logements du deuxième élément (30) est inférieure ou égale à la moitié de la largeur de la bague (11) du premier élément (10).

10. Raccord selon l'une quelconque des Revendications précédentes, caractérisé en ce que la bague (11) du premier élément (10) comporte, sur sa face (14) opposée à celle destinée à venir en contact de l'embase (31) du deuxième élément (30), une nervure (16) prolongée par une collerette radiale (17) pour sa fixation sur l'appareillage pour stomie.

11. Raccord selon l'une quelconque des Revendications précédentes, caractérisé en ce que la bague (11) du premier élément (10) est à section droite sensiblement rectangulaire.

12. Raccord selon l'une quelconque des Revendications précédentes, caractérisé en ce que la bague (11) du premier élément (10) est à section droite sensiblement en forme de U et loge un joint torique (18).

13. Raccord selon l'une quelconque des Revendications précédentes, caractérisé en ce que les contours interne (12) et externe (13) de la bague (11) du premier élément (10) comporte chacun une découpe concave qui ménage avec la face (15) de cette bague destinée à être en contact avec l'embase (31) de l'élément (30) deux lèvres (25, 26) propres à assurer, par déformation élastique, l'étanchéité entre les deux éléments (10, 30) du raccord.

14. Raccord selon l'une quelconque des Revendications précédentes, caractérisé en ce que la bague (11) du premier élément (10) et l'embase (31) du deuxième élément (30) sont annulaires.

15. Raccord selon l'une quelconque des Revendications précédentes, caractérisé en ce qu'au moins l'un des deux éléments du raccord est muni de moyens de préhension (85, 86) propres à faciliter l'assemblage de ces deux éléments.

16. Raccord selon l'une quelconque des Revendications 10 à 15, caractérisé en ce que la collerette du premier élément et/ou l'embase du deuxième élément comporte un ou plusieurs plots (90) propres à prévenir tout déplacement radial involontaire du premier élément par rapport au deuxième élément dans leur condition d'assemblage.

17. Appareillage pour stomie comprenant un porte-poches (70) destiné à être fixé autour d'une ouverture artificielle du corps d'un utilisateur, ainsi qu'une poche (80) pour le recueil des matières corporelles propre à être assemblée de manière amovible au porte-poches, caractérisé en ce qu'il comprend un raccord selon l'une quelconque des Revendications 1 à 15.
